# EUROPÄISCHE PATENTSCHRIFT

(11) **EP 3 024 521 B1**
(45) Veröffentlichungstag und Bekanntmachung des Hinweises auf die Patenterteilung: **17.10.2018**
(21) Anmeldenummer: 14744064.8
(22) Anmeldetag: 23.07.2014
(51) Int. Cl.: A61M 11/00, A61M 15/00, A61J 3/00, B65D 41/06

(54) **WECHSELKARTUSCHE**
REPLACEMENT CARTRIDGE
CARTOUCHE INTERCHANGEABLE

(30) Priorität: 24.07.2013 DE 102013214471
(43) Veröffentlichungstag der Anmeldung: 01.06.2016
(73) Patentinhaber: Medic Activ Vertriebs GmbH, 82031 Grünwald (DE)
(72) Erfinder: BRUNNER, Peter, 85737 Ismaning (DE); LINDENA, Bernd Heiko, A-6363 Westendorf (AT)
(74) Vertreter: Dilg, Haeusler, Schindelmann Patentanwaltsgesellschaft mbH
(86) Internationale Anmeldenummer: PCT/EP2014/065859
(87) Internationale Veröffentlichungsnummer: WO 2015/011210

(56) Entgegenhaltungen:
- WO-A1-2011/082838
- WO-A2-2013/030117
- US-A- 4 434 903
- US-A1- 2005 242 055

## Beschreibung

Die vorliegende Erfindung betrifft eine Wechselkartusche zur Erzeugung eines Aerosols mit wenigstens einem Bodenelement und wenigstens einem Deckelelement, wobei in dem Bodenelement und/oder Deckelelement ein Reservoir für eine erste, insbesondere flüssige, Aerosolkomponente gebildet ist.

Wechselkartuschen der voranstehend genannten Art werden unter anderem in Verneblern, Inhalatoren oder Kabinen eingesetzt, um die Atemwege oder Körper von Lebewesen einem Aerosol auszusetzen. Dies kann sowohl zu therapeutischen als auch zu Desinfektionszwecken geschehen.

In der WO 2011/082838 wird eine Wechselkartusche zur Erzeugung eines Aerosols beschrieben, die aus mindestens zwei Bauteilen besteht, die nach dem Einfüllen eines zu zerstäubenden Fluids miteinander verbindbar sind. Dies kann beispielsweise über eine Steckverbindung oder eine Schraubverbindung geschehen.

Zur Herstellung einer Schraubverbindung müssen die Bauteile genau zueinander ausgerichtet werden und in dieser Stellung gegeneinander verdreht werden. Dies hat einen erhöhten Zeitbedarf beim Verschließen der Kartusche nach dem Einfüllen des zu zerstäubenden Fluids zur Folge. Außerdem ist der Prozess im Hinblick auf große Stückzahlen schlecht automatisierbar. Eine einfache Steckverbindung bietet nur geringen Schutz gegen ein unbeabsichtigtes Öffnen der Kartusche.

Bevorzugt wird gemäß der WO 2011/082838 daher eine unlösbare Verbindung der beiden Bauteile durch Verkleben und insbesondere Verschweißen. Es hat sich gezeigt, dass nach längerer Lagerung von verklebten oder verschweißten Kartuschen Undichtigkeiten auftreten können.

In der DE 10 2011 079 810 wird eine Ampulle zum Einsetzen in einen Aerosolerzeuger, um eine in der Ampulle enthaltene Substanz zu vernebeln, beschrieben. Die Ampulle umfasst einen Behälterabschnitt, der eine Kammer zur Aufnahme zumindest eines Bestandteils der Substanz bildet, und einen Schnittstellenabschnitt, der zur Aufnahme und Festlegung der Ampulle in dem Aufnahmeabschnitt des Aerosolerzeugers ausgestaltet ist, mittels welchem Aerosolerzeuger dann aus der Substanz ein Aerosol erzeugt wird.

In der DE 87 04 273 wird eine Vorrichtung zum Versprühen von Stoffen bestehend aus einem Druckbehälter mit einer durch einen Deckel druckdicht verschließbaren Öffnung zum Einbringen des zu versprühenden Stoffes in den Behälterinnenraum, mit einer außerhalb des Behälters angeordneten Sprühdüse, die über einen durch ein Sprühventil zu öffnenden Durchlass mit dem Behälterinnenraum verbunden ist, sowie mit Mitteln zum Einbringen eines Druckmediums in den Behälterinnenraum, beschrieben.

In der DE 199 40 713 wird eine Kartusche für Flüssigkeiten beschrieben. Die Kartusche ist ein dreischaliger Behälter, der aus einem die Flüssigkeit enthaltenden kollabierbaren Beutel, einem formstabilen Behälter und einer steifen Metallhülse bestehen kann. Die Kartusche kann mit einem Entnahmegerät lösbar verbunden werden, welcher als separater Zerstäuber für die Erzeugung eines inhalierbaren Aerosols zur Behandlung von Krankheiten dient.

Hiervon ausgehend lag der vorliegenden Erfindung die Aufgabe zugrunde, eine Kartusche der oben genannten Art anzugeben, die sich einfacher herstellen lässt und einen besseren Schutz gegen Undichtigkeiten aufweist.

Erfindungsgemäß wurde die Aufgabe durch eine Wechselkartusche zur Erzeugung eines Aerosols mit wenigstens einem Bodenelement und mit wenigstens einem Deckelelement, wobei in dem Bodenelement und/oder Deckelelement ein Reservoir für eine erste, insbesondere flüssige, Aerosolkomponente, gebildet ist, wobei in der Wechselkartusche eine Zerstäubungsdüse gebildet ist und wobei Bodenelement und Deckelelement mittels eines Bajonettverschlusses miteinander verbunden sind, gelöst.

Ein Vorteil der oben beschriebenen Wechselkartusche mag sein, dass die Zerstäubungsdüse innerhalb der Wechselkartusche gebildet ist, so dass die Zerstäubungsdüse gegen Schmutz und Beschädigung geschützt ist. Somit mag eine gleichmäßigere und sicherere Zerstäubung erreicht werden.

Durch die Translationsbewegung aufeinander zu können das Bodenelement und das Deckelelement auf einfache Weise für die nachfolgende Drehbewegung ausgerichtet werden. Zum Schließen eines Bajonettverschlusses ist zudem nur ein zeitsparender geringer Drehwinkel erforderlich.

Weiter kann es die Verwendung eines Bajonettverschlusses im Unterscheid zu einem Schraubverschluss erlauben, größere Abmessungstoleranzen bei der Herstellung der Wechselkartusche vorzusehen. Es kann somit z.B. auf kostengünstigere Produktionsprozesse, beispielsweise Spritzgießen, zurückgegriffen werden.

Der Bajonettverschluss kann beispielsweise durch Vorsprünge gebildet werden, die von entsprechenden Haken hintergriffen werden. Die Wahl von drei Paaren aus Vorsprüngen und Haken für den Bajonettverschluss kann einen geringen Drehwinkel bei gleichzeitig unproblematischem Zusammenführen des Bodenelements und des Deckelelements ermöglichen.

Nach einem Ausführungsbeispiel weist das Bodenelement und/oder das Deckelelement eine Zentrierfläche auf. Die Zentrierfläche kann das vor der Drehbewegung notwendige Zusammenfügen des Bodenelementes und des Deckelements vereinfachen.

Gemäß der Erfindung weist die Wechselkartusche wenigstens einen dem Bajonettverschluss zugeordneten Rastvorsprung und wenigstens eine dem Bajonettverschluss zugeordnete Rastaufnahme auf, wobei das Bodenelement und das Deckelelement mittels des Rastvorsprungs und der Rastaufnahme miteinander verrastbar sind. Durch ein Verrasten kann eine für eine sichere und dauerhaft Verbindung von dem Bodenelement und dem Deckelelement ausreichende Drehbewegung von verschließenden Personen oder Maschinen erkannt werden. Zudem kann mittels Verrasten einem unbeabsichtigten Öffnen der Wechselkartusche entgegengewirkt werden.

Gemäß der Erfindung sind der Rastvorsprung und/oder die Rastaufnahme so ausgebildet, dass Bodenelement und Deckelement nur einmal miteinander verrastbar sind. Auf diese Weise kann vermieden werden, dass die Wechselkartusche von Unbefugten geöffnet und mit einer ungeeigneten zweiten Aerosolkomponente wiederbefüllt wird.

Dazu weisen der Rastvorsprung und/oder die Rastaufnahme eine Sollbruchstelle auf, die beim Öffnen der Verrastung zur, insbesondere sichtbaren, Zerstörung des Rastvorsprungs und/oder der Rastaufnahme führt. Durch das Vorsehen einer Sollbruchstelle kann es ermöglicht werden, dass die Wechselkartusche nach dem Verschließen zwar wieder geöffnet, aber nicht nochmals sicher verschlossen werden kann. Ein Öffnen der Wechselkartusche kann es beispielsweise erlauben, Reste der zweiten Aerosolkomponente nach dem Gebrauch der Wechselkartusche zu entnehmen und einer von den übrigen Bestandteilen der Wechselkartusche getrennten Entsorgung zuzuführen.

Die Sollbruchstelle kann insbesondere nur am Bodenelement bzw. Deckelement vorgesehen sein. In diesem Fall kann das unzerstörte Deckelelement bzw. Bodenelement nach einer ggf. erforderlichen Reinigung und/oder Sterilisation für die Herstellung einer neuen Wechselkartusche ggf. wiederverwendet werden und auf diese Weise zu einem nachhaltigen Ressourcenverbrauch beigetragen werden. Durch die Sichtbarkeit der zerstörten Sollbruchstelle nach der Öffnung kann es für Verwender der Wechselkartusche ermöglicht werden, diese sicher von originalverpackten Wechselkartuschen zu unterscheiden.

Weiter weist gemäß einer Ausgestaltung die Wechselkartusche einen zwischen Bodenelement und Deckelelement angeordneten Dichtring auf. Mit dem Dichtring kann ein fluiddichter Einschluss der zweiten Aerosolkomponente in der Wechselkartusche gewährleistet werden. Der Dichtring kann insbesondere axial zwischen dem Bodenelement und Deckelelement angeordnet sein und eine Stirnfläche des Bodenelements und eine Stirnfläche des Deckelements gegeneinander abdichten. Ebenso kann der Dichtring radial zwischen dem Bodenelement und Deckelelement angeordnet sein und eine Außenumfangsfläche des Bodenelements bzw. Deckelements gegen eine Innenumfangsfläche des Deckelelements bzw. Bodenelements abdichten, so dass auch bei geringem, durch den Bajonettverschluss in axialer Richtung ausgeübtem Druck eine gute Abdichtung gewährleistet werden kann. Ebenso kann der Dichtring vor dem Zusammenfügen von Bodenelement und Deckelement verliersicher auf die Außenumfangsfläche des Bodenelements bzw. Deckelelements geschoben werden.

Darüber hinaus umfasst gemäß einer Weiterbildung die Wechselkartusche eine Druckgasflasche, insbesondere eine Druckgaskartusche. Eine Wechselkartusche, die eine Druckgasflasche, insbesondere eine Druckgaskartusche, aufweist, kann autark eingesetzt werden. Insbesondere kann auf einen separaten Kompressor zur Erzeugung eines Druckgases zum Ausbringen des Aerosols verzichtet werden. Dies kann insbesondere in feuchten Umgebungen sinnvoll sein, in denen elektrische Anlagen besonderen Sicherheitsstandards genügen müssen.

Die Gasflasche kann mit einem Ventil versehen sein, so dass das Ausbringen des Aerosols unterbrochen und später wieder aufgenommen werden kann. Es kann jedoch auch eine Gasflasche ohne Ventil verwendet werden, so dass für jede Anwendung eine genau definierte Menge an Gas bereitgestellt werden kann.

Die Druckgasflasche kann lösbar mit dem Bodenelement und/oder Deckelelement verbunden sein. Auf diese Weise kann die Art des zum Ausbringen der im Reservoir befindlichen ersten Aerosolkomponente verwendeten Gases in Abhängigkeit der vorgesehenen Anwendung gewechselt werden. Beispielsweise kann vorgesehen sein, die erste Aerosolkomponente normalerweise mit kostengünstig verfügbarem Stickstoff auszubringen und in besonderen Fällen auf Sauerstoff zurückzugreifen. Ebenso kann es ermöglicht werden, den Inhalt einer Druckgasflasche mit unterschiedlichen ersten Aerosolkomponenten zu verwenden. Dies kann sich insbesondere dann anbieten, wenn unterschiedliche erste Aerosolkomponenten mit der gleichen Gasart ausgebracht werden sollen. Zur Herstellung der lösbaren Verbindung kann beispielsweise die Gasflasche in das Bodenelement und/oder das Deckelement eingeschraubt werden. Diese Art der Verbindung kann den Vorteil haben, dass kostengünstig verfügbare, handelsübliche Druckgasflaschen verwendet werden können. In Betracht können insbesondere Druckgasflaschen kommen, die normalerweise zum Aufblasen von Rettungsmitteln, d.h. Schwimmwesten oder Rettungsinseln, vorgesehen sind. Ebenso kann die Verbindung auch mittels eines Bajonettverschlusses hergestellt werden. Auf die mit dieser Art der Verbindung einhergehenden Vorteile wurde bereits oben in Bezug auf die Verbindung Bodenelement/Deckelelement hingewiesen.

Es ist auch denkbar, die Druckgasflasche unlösbar mit dem Bodenelement und/oder Deckelelement zu verbinden. In diesem Fall kann gewährleistet werden, dass immer eine bestimmte Kombination von Gas und erster Aerosolkomponente zur Anwendung gelangt und die Komponenten nicht in unzulässiger, möglicherweise gesundheitsgefährdender Weise gemischt werden. Weiter kann diese Ausgestaltung mit unlösbarer Verbindung die Nutzung der Wechselkartusche vereinfachen, indem für eine neue Anwendung nur der Austausch eine einzigen Elements, d.h. der Wechselkartusche, ausreichen kann.

Ferner sieht die Ausgestaltung der Wechselkartusche vor, dass die Druckgasflasche ein wohlriechendes und/oder desinfizierendes und/oder therapeutisch wirksames Gas, insbesondere Sauerstoff, enthält. Mit anderen Worten wird das Gas nicht zur als passive zum Ausbringen und Zerstäubung der ersten Aerosolkomponente verwendet, sondern es trägt aktiv Eine derartige Ausgestaltung kann es erlauben, eine Abstimmung zwischen der ersten Aerosolkomponente und dem Gas zur Erreichung eines hohen Therapieerfolges vorzunehmen.

In der Zeichnung zeigt
- Figur 1: ein Bodenelement, ein Deckelelement, einen Dichtring und einen Düseneinsatz;
- Figur 2: ein Deckelelement, einen Dichtring und ein Bodenelement;
- Figur 3: ein Deckelelement und ein Bodenelement;
- Figur 4: eine Wechselkartusche;
- Figur 5: die Unterseite eines Bodenelements;
- Figur 6: die Oberseite eines Deckelelementes;
- Figur 7: die Unterseite eines Deckelementes; und
- Figur 8: einen Ausschnitt eines Bodenelementes.

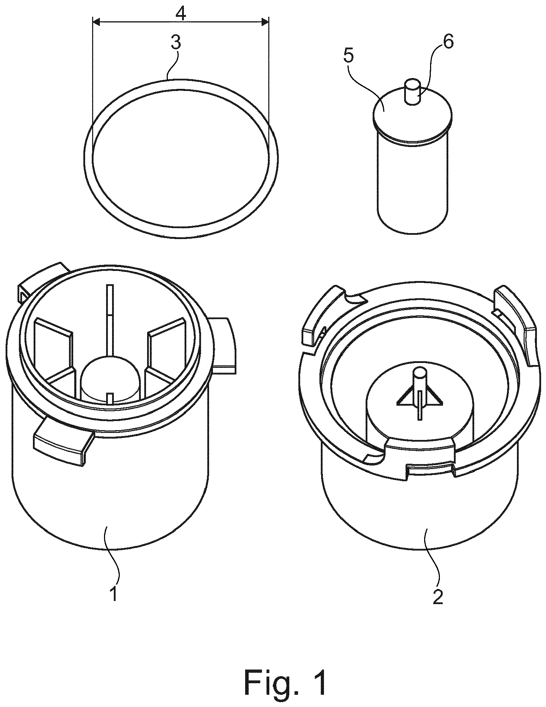

In der Figur 1 ist ein Bodenelement 1, ein Deckelelement 2, ein Dichtring 3 mit einem Innendurchmesser 4 und ein Düseneinsatz 5 abgebildet. Das Bodenelement 1, das Deckelelement 2 und der Düseneinsatz 5 sind als Spritzgussteile ausgebildet. Im Spritzgussverfahren lassen sich insbesondere Kunststoffe auf kostengünstige Weise in die benötigte Form bringen.

Die Abmessungen des Düseneinsatzes 5, insbesondere der Düsenöffnung 6, können einen besonders großen Einfluss auf die Größe der Aerosolpartikel oder -tröpfchen haben, so dass es grundsätzlich auch denkbar ist es, für den Düseneinsatz 5 ein andere Herstellungsverfahren und/oder Material vorzusehen mit dem geringere Fertigungstoleranzen eingehalten werden können. Durch die geometrisch einfache Form des Düseneinsatzes 5 muss ein Wechsel des Herstellungsverfahrens für den Düseneinsatz 5 auch nicht zu höheren Kosten führen.

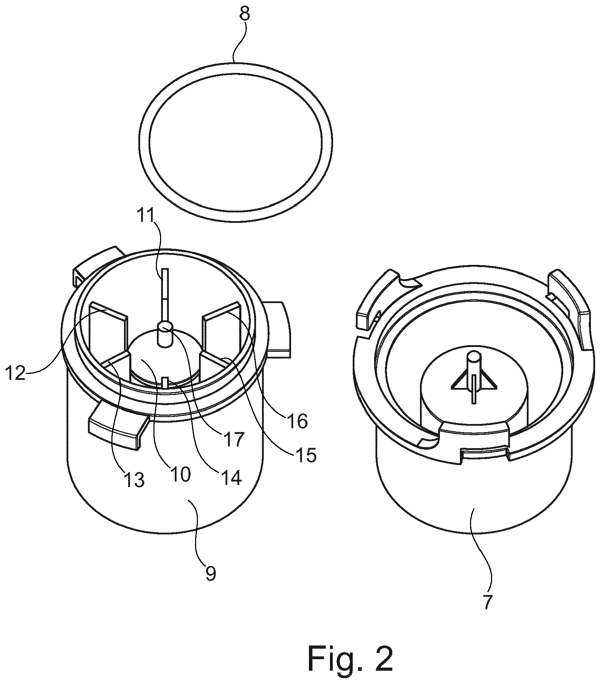

Die Figur 2 zeigt ein Deckelelement 7, einen Dichtring 8 und ein Bodenelement 9, in welches ein Düseneinsatz 10 eingesetzt wurde. Der Düseneinsatz wird durch Rippen 11, 11, 12, 13, 14, 15, 16 in radialer Richtung fixiert. Die Rippen 11, 12, 13, 14, 15, 16 können zudem zur Aussteifung des Bodenelements 9 beitragen. Der Düseneinsatz 10 ist in axialer Richtung beweglich und kann beispielsweise mit einem anderen (hier nicht dargestellten) Düseneinsatz aus einem anderen Material und/oder mit einer von der Düsenöffnung 17 in Form und/oder Größe abweichenden Düsenöffnung ausgetauscht werden.

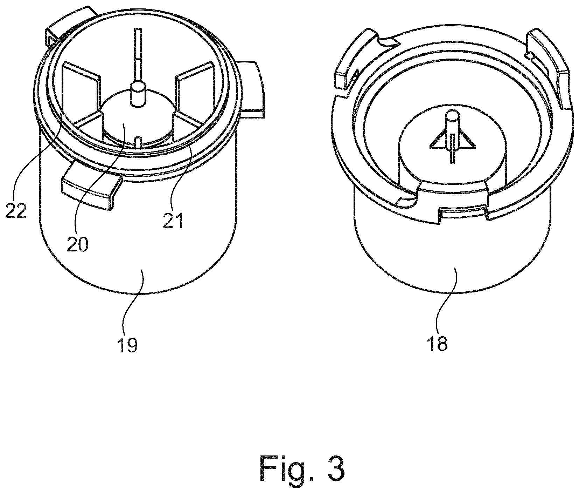

In der Figur 3 sind ein Deckelelement 18 und ein Bodenelement 19 dargestellt. In das Bodenelement 19 wurde ein Düseneinsatz 20 eingesetzt und ein Dichtring 21 über eine Außenumfangsfläche 22 des Bodenelements geschoben.

Der Dichtring 21 kann sowohl vor als auch nach dem Einsetzen des Düseneinsatz 20 über die Außenumfangsfläche 22 geschoben werden. Der Innendurchmesser eines elastischen Dichtrings 21 kann beispielsweise geringer gewählt werden als der die Außenumfangsfläche 22, so dass nach dem Überschieben der Dichtring 21 verliersicher von der Außenumfangsfläche 22 gehalten wird. Bei einem verliersicher gehaltenen Dichtring 21 kann es sich anbieten, diesen über die Außenumfangsfläche 22 zu schieben, bevor der Düseneinsatz 20 eingesetzt wird, um ein Herausfallen des Düseneinsatzes 20 während der Manipulation des Bodenelementes 19, beispielsweise beim Anbringen des Dichtringes 21, oder beim Transport des Bodenelementes zu vermeiden.

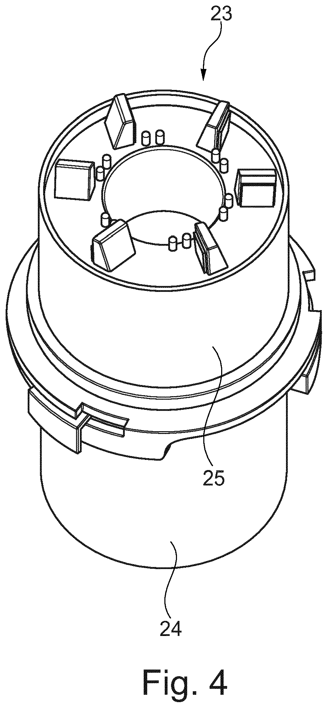

Die Figur 4 zeigt eine Wechselkartusche 23 mit einem Bodenelement 24 und einem Deckelelement 25, die mittels eines Bajonettverschlusses miteinander verbunden sind.

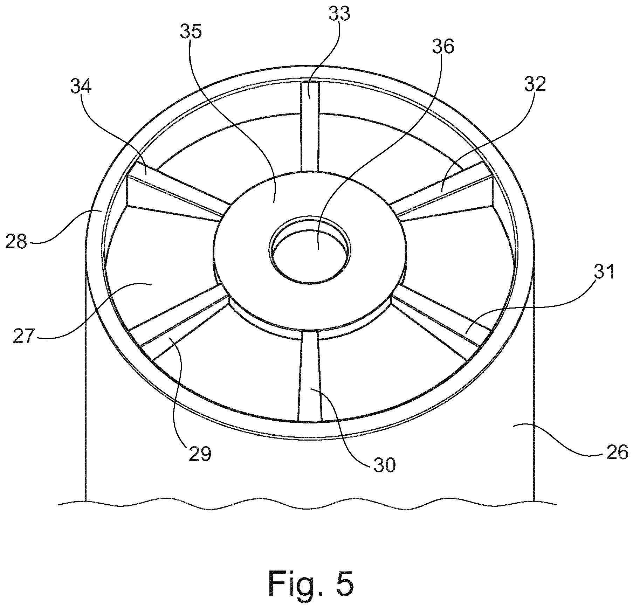

In der Figur 5 ist die Unterseite eines Bodenelements 26 dargestellt. Der Boden 27 des Bodenelementes 26 ist gewölbt. Durch die Wölbung des Bodens 27 kann die nicht ausbringbare Menge der in einem Reservoir des Bodenelementes befindlichen Aerosolkomponente verringert werden. Dies kann zu einer Reduktion des Ressourcenverbrauches und einer Kostenminderung beitragen. Das Bodenelement 26 ist mit vom Zylindermantel 28 ausgehenden Rippen 29, 30, 31, 32, 33, 34 ausgesteift. Die Rippen 29, 30, 31, 32, 33, 34 können sich bis in den Innenraum des Bodenelementes 26 fortsetzen und entsprechend den in der Figur 2 dargestellten Rippen 11 , 12, 13, 14, 16 zur radialen Fixierung eines Düseneinsatzes dienen. Im radialen Zentrum des Bodenelementes 26 ist ein Hohlzylinder 35 ausgebildet, der mittels einer Abdeckung 36 verschlossen ist. In dem gezeigten Ausführungsbeispiel ist die Abdeckung 36 einstückig mit dem Boden 27 und den restlichen Elementen des Bodenelementes 26 ausgeführt. Der Hohlzylinder 35 kann grundsätzlich jedoch auch mit einer Folie, insbesondere einer Aluminium- oder Kunststofffolie abgedeckt sein. Der Hohlzylinder 35 kann in einem hier nicht gezeigten Ausführungsbeispiel ein Innengewinde aufweisen, in das eine Gasflasche eingeschraubt werden kann.

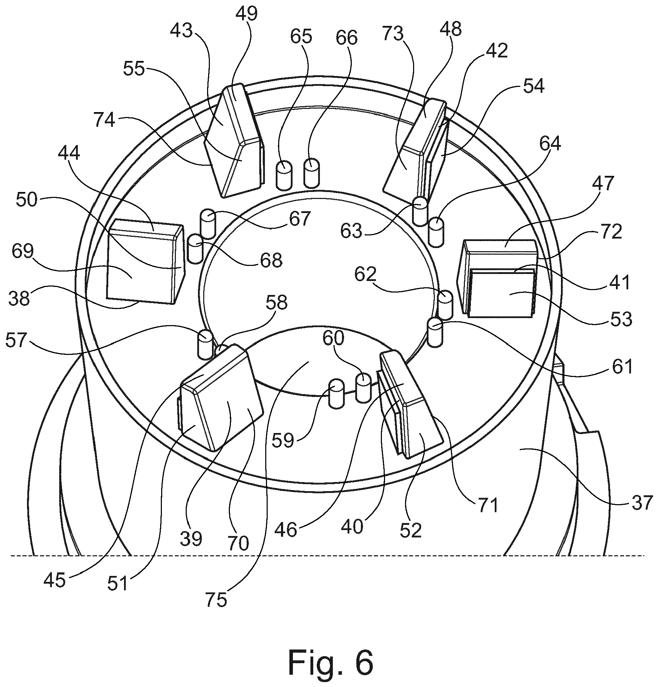

Die Figur 6 zeigt die Oberseite eines Deckelelementes 37. An der Oberseite des Deckelelementes 37 sind Öffnungshilfen 38, 39, 40, 41, 42, 43 bereitgestellt. Beim Einsetzen einer mit dem Deckelelement 37 versehenen Wechselkartusche in die zugehörige Vorrichtung kommen die Öffnungshilfen 38, 39, 40, 41, 42, 43 an ihrem oberen Ende 44, 45, 46, 47, 48, 49 zunächst mit einer entsprechend ausgebildeten Kontaktfläche der Vorrichtung in Kontakt und knicken anschließend um die Knicklinien 50, 51, 52, 53, 54, 55 ab, bevor die Wechselkartusche mit dem Zylindermantel 56 und den Anschlagsstiften 57 bis 68 an der Kontaktfläche der Vorrichtung anschlägt. Die Öffnungshilfen 38, 39, 40, 41, 42, 43 reißen dabei entlang der Sollbruchstellen 69, 70, 71, 72, 73, 74 ab, so dass Öffnungen zu dem vom Deckelement 37 umschlossenen Raum gebildet werden, durch die innerhalb der Wechselkartusche erzeugtes Aerosol in die Vorrichtung ausgebracht werden kann. Der an der Oberseite des Deckelelementes 37 gebildete Hohlzylinder 75 kann während dessen zur Zentrierung der Wechselkartusche in der Vorrichtung dienen.

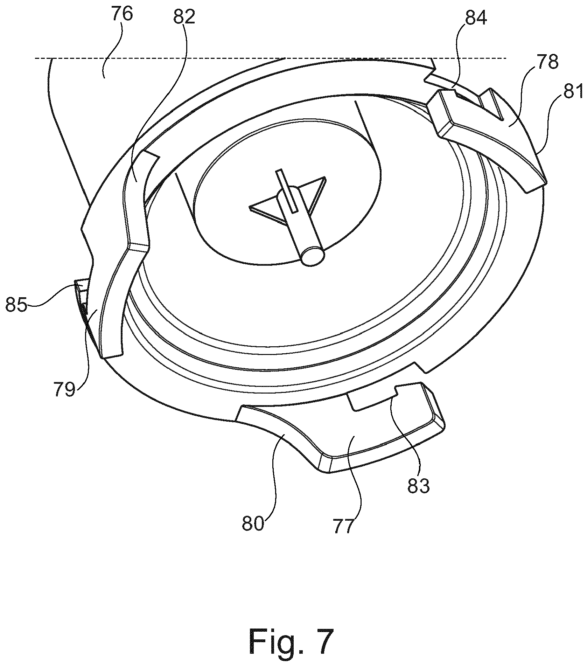

In der Figur 7 ist die Unterseite eines Deckelelementes 76 abgebildet. Das Deckelelement weist drei Haken 77, 78, 79 auf, die mit korrespondierenden Vorsprüngen eines hier nicht dargestellten Bodenelementes zur Bildung eines Bajonettverschlusses zusammenwirken können. Die Haken können ebenso am Bodenelement ausgebildet sein und mit Vorsprüngen eines Deckelelementes zusammenwirken. Die Haken 77, 78, 79 weisen jeweils eine Abgleitfläche 80, 81, 82 auf, an der die korrespondierenden Vorsprünge des Bodenelementes beim Zusammenfügen von Bodenelement und Deckelelement 76 abgleiten können. An den Haken 77, 78, 79 sind weiter Rastvorsprünge 83, 84, 85 ausgebildet, mit deren Hilfe das Deckelelement 76 mit einem korrespondierenden Bodenelement verrastbar ist.

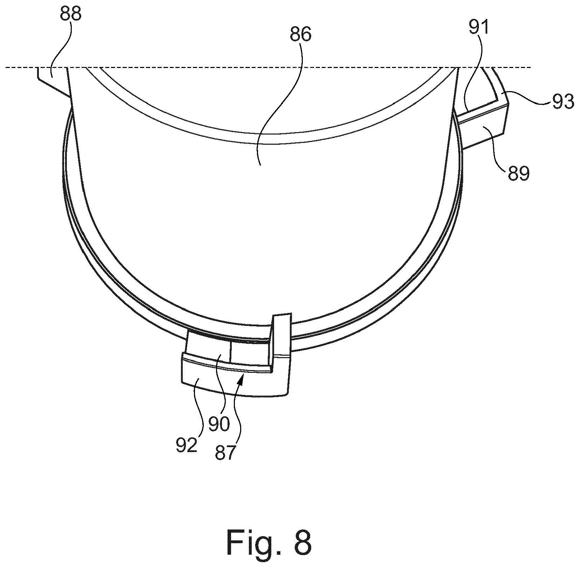

Die Figur 8 zeigt einen Ausschnitt eines Bodenelementes 86 mit drei Vorsprüngen 87, 88, 89, welche mit Haken eines Deckelelementes zur Bildung eines Bajonettverschlusses zusammenwirken können. Der Vorsprung 87 weist ebenso wie die anderen Vorsprünge 88, 89 eine Rastaufnahme 90 auf, über die das Bodenelemente 86 mit korrespondierenden Rastvorsprüngen eines Deckelelementes verrastbar ist. Das Bodenelement 86 ist dafür vorgesehen, nur einmal mit einem korrespondierenden Deckelelement verrastet zu werden, um sicherzustellen, dass die Wechselkartusche nur durch dazu befugte Personen mit der ersten Aerosolkomponente befüllt werden kann. Insbesondere bei medizinischen Verwendungen der Wechselkartusche kann es darauf ankommen, dass beim Befüllen der Wechselkartusche sterile Bedingungen herrschen. Ebenso wird für medizinische Verwendungen typischerweise eine manipulationssichere Wechselkartusche gefordert, deren medizinisch wirksame erste Aerosolkomponente nicht durch ein nicht wirksames oder ggf. sogar schädliches Mittel ausgetauscht werden kann. Die Rastaufnahmen 90, 91 sind in radialer Richtung von einem Schutzsteg 92, 93 geschützt. Im miteinander verbundenen Zustand von Bodenelement 86 und zugehörigem Deckelelement kann die Verrastung auf diese Weise vor Manipulationen zur Öffnung der Wechselkartusche geschützt werden.

## Patentansprüche

1. Wechselkartusche zur Erzeugung eines Aerosols
mit wenigstens einem Bodenelement (1) und
mit wenigstens einem Deckelelement (2),
wobei in dem Bodenelement (1) und/oder Deckelelement (2) ein Reservoir für eine erste, insbesondere flüssige, Aerosolkomponente, gebildet ist,
wobei in der Wechselkartusche eine Zerstäubungsdüse gebildet ist, **dadurch gekennzeichnet, dass**
Bodenelement (1) und Deckelelement (2) mittels eines Bajonettverschlusses miteinander verbunden sind;
dass die Wechselkartusche wenigstens einen dem Bajonettverschluss zugeordneten Rastvorsprung (83) und wenigstens eine dem Bajonettverschluss zugeordnete Rastaufnahme (90) aufweist, wobei das Bodenelement (1) und das Deckelelement (2) mittels des Rastvorsprungs (83) und der Rastaufnahme (90) miteinander verrastbar sind; und
dass der Rastvorsprung (83) und/oder die Rastaufnahme (90) eine Sollbruchstelle aufweisen, die beim Öffnen der Verrastung zur, insbesondere sichtbaren, Zerstörung des Rastvorsprung und/oder der Rastaufnahme führt.

2. Wechselkartusche nach Patentanspruch 1,
**dadurch gekennzeichnet,**
**dass** der Bajonettverschluss mindestens einen Vorsprung (87) und mindestens einen Haken (77) aufweist, und
**dass** der Haken (77) eine Abgleitfläche (80) aufweist, an welcher der Vorsprung (87) beim Zusammenführen des Bodenelements (1) und des Deckelelements (2) abgleiten kann.

3. Wechselkartusche nach einem der Patentsprüche 1 oder 2,
**dadurch gekennzeichnet,**
**dass** das Bodenelement (1) und/oder das Deckelelement (2) eine Zentrierfläche aufweist.

4. Wechselkartusche nach einem der Patentansprüche 1 bis 3,
**dadurch gekennzeichnet,**
**dass** der Rastvorsprung (83) und/oder die Restaufnahme (90) eine Abdeckung (92) aufweisen.

5. Wechselkartusche nach einem der Patentansprüche 1 bis 4,
**dadurch gekennzeichnet,**
**dass** die Wechselkartusche einen zwischen Bodenelement (1) und Deckelelement (2) angeordneten Dichtring (3) aufweist.

6. Wechselkartusche nach einem der Patentansprüche 1 bis 5,
**dadurch gekennzeichnet,**
**dass** die Kartusche eine lösbar oder unlösbar mit dem Bodenelement (1) und/oder Deckelelement (2) verbundene Druckgasflasche, insbesondere Druckgaskartusche, umfasst.

7. Wechselkartusche nach einem der Patentansprüche 1 bis 6,
**dadurch gekennzeichnet,**
**dass** die Druckgasflasche ein wohlriechendes und/oder desinfizierendes und/oder therapeutisch wirksames Gas, insbesondere Sauerstoff, enthält.

## Claims

1. Exchangeable cartridge for generating an aerosol,
with at least one bottom element (1) and
with at least one cover element (2),
wherein in the bottom element (1) and/or cover element (2) a reservoir is formed for a first, in particular liquid, aerosol component,
wherein in the exchangeable cartridge a nebulizer nozzle is formed, **characterized in that**
the bottom element (1) and the cover element (2) are connected to each other by a bayonet catch;
that the exchangeable cartridge comprises at least one engagement protrusion (83) assigned to the bayonet catch and at least one engagement accommodation (90) assigned to the bayonet catch, wherein the bottom element (1) and the cover element (2) are engageable with each other by the engagement protrusion (83) and the engagement accommodation (90); and
that the engagement protrusion (83) and/or the engagement accommodation (90) comprises a predetermined breaking location which, when opening the engagement, leads to a destruction, in particular a visible destruction, of the engagement protrusion and/or the engagement accommodation.

2. Exchangeable cartridge according to claim 1,
**characterized in that**
the bayonet catch comprises at least one protrusion (87) and at least one hook (77), and
that the hook (77) comprises a sliding surface (80) at which the protrusion (87) can slide when bringing together the bottom element (1) and the cover element (2).

3. Exchangeable cartridge according to any one of claims 1 or 2,
**characterized in that**
the bottom element (1) and/or the cover element (2) comprises a centering surface.

4. Exchangeable cartridge according to any one of claims 1 to 3,
**characterized in that**
the engagement protrusion (83) and/or the engagement accommodation (90) comprise a coverage (92).

5. Exchangeable cartridge according to any one of claims 1 to 4,
**characterized in that**
the exchangeable cartridge comprises a sealing ring (3) arranged between the bottom element (1) and the cover element (2).

6. Exchangeable cartridge according to any one of claims 1 to 5,
**characterized in that**
the cartridge comprises a pressure gas bottle, in particular a pressure gas cartridge, which is detachably or non-detachably connected with the bottom element (1) and/or the cover element (2).

7. Exchangeable cartridge according to any one of claims 1 to 6,
**characterized in that**
the pressure gas bottle contains a fragrant and/or disinfectant and/or therapeutically effective gas, in particular oxygen.

## Revendications

1. Cartouche interchangeable pour générer un aérosol
avec au moins un élément de fond (1) et
avec au moins un élément formant couvercle (2),
un réservoir pour un premier composant d'aérosol, en particulier liquide, étant formé dans l'élément de fond (1) et/ou l'élément formant couvercle (2),
une buse de pulvérisation étant formée dans la cartouche interchangeable, **caractérisée en ce que**
l'élément de fond (1) et l'élément formant couvercle (2) sont reliés l'un à l'autre au moyen d'une fermeture à baïonnette ;
que la cartouche interchangeable présente au moins une partie faisant saillie d'enclenchement (83) associée à la fermeture à baïonnette et au moins un logement d'enclenchement (90) associé à la fermeture à baïonnette, l'élément de fond (1) et l'élément formant couvercle (2) pouvant être enclenchés l'un avec l'autre au moyen de la partie faisant saillie d'enclenchement (83) et du logement d'enclenchement (90) ; et
que la partie faisant saillie d'enclenchement (83) et/ou le logement d'enclenchement (90) présentent un point de rupture théorique, qui conduit, lors de l'ouverture de l'enclenchement, à la destruction, en particulier visible, de la partie faisant saillie d'enclenchement et/ou du logement d'enclenchement.

2. Cartouche interchangeable selon la revendication 1, **caractérisée en ce**
**que** la fermeture à baïonnette présente au moins une partie faisant saillie (87) et au moins un crochet (77), et
**que** le crochet (77) présente une surface de glissement (80), au niveau de laquelle la partie faisant saillie (87) peut glisser lors du rassemblement de l'élément de fond (1) et de l'élément formant couvercle (2) .

3. Cartouche interchangeable selon l'une quelconque des revendications 1 ou 2, **caractérisée en ce**
**que** l'élément de fond (1) et/ou l'élément formant couvercle (2) présentent une surface de centrage.

4. Cartouche interchangeable selon l'une quelconque des revendications 1 à 3, **caractérisée en ce**
**que** la partie faisant saillie d'enclenchement (83) et/ou le logement d'enclenchement (90) présentent un recouvrement (92).

5. Cartouche interchangeable selon l'une quelconque des revendications 1 à 4, **caractérisée en ce**
**que** la cartouche interchangeable présente une bague d'étanchéité (3) disposée entre l'élément de fond (1) et l'élément formant couvercle (2).

6. Cartouche interchangeable selon l'une quelconque des revendications 1 à 5, **caractérisée en ce**
**que** la cartouche comprend une bouteille de gaz sous pression, en particulier une cartouche de gaz sous pression, reliée de manière amovible ou inamovible à l'élément de fond (1) et/ou à l'élément formant couvercle (2) .

7. Cartouche interchangeable selon l'une quelconque des revendications 1 à 6, **caractérisée en ce**
**que** la bouteille de gaz sous pression contient un gaz odorant et/ou désinfectant et/ou à action thérapeutique, en particulier de l'oxygène.
